(19) Europäisches Patentamt
European Patent Office
Office européen des brevets

(11) Publication number: **0 219 343 A2**

(12) **EUROPEAN PATENT APPLICATION**

(21) Application number: 86307934.9

(22) Date of filing: 14.10.86

(51) Int.Cl.[4]: C 07 C 131/00
C 07 D 307/94, C 07 D 209/5-4
A 01 N 37/08, A 01 N 37/18
A 01 N 43/08, A 01 N 43/38

(30) Priority: 14.10.85 AU 2895/85

(43) Date of publication of application: 22.04.87 Bulletin 87/17

(84) Designated Contracting States: AT BE CH DE ES FR GB GR IT LI LU NL SE

(71) Applicant: Dunlena Pty. Limited
168 Walker Street
North Sydney New South Wales(AU)

(72) Inventor: Liepa, Andris Juris
5 Wellwood Square
Wheelers Hill, Victoria(AU)

(72) Inventor: Winzenberg, Kevin Norman
Flat 2, 28 Tivoli Road
South Yarra, Victoria(AU)

(74) Representative: Lawrence, Peter Robin Broughton
GILL JENNINGS & EVERY 53-64 Chancery Lane
London WC2A 1HN(GB)

(54) **Cyclohexane-1,3-dione derivatives having a herbicidal activity.**

(57) Compounds of the formula (2), including all isomeric and/or tautomeric forms.

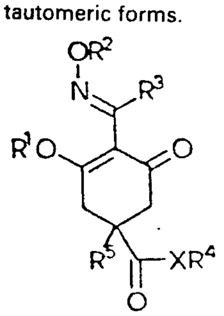

(2)

where X is O or $NR^6$, $R^1$ is an organic group, hydrogen or an inorganic cation, $R^2$ to $R^6$ are independently organic groups or hydrogen are disclosed. The presence of the group $R^5$ gives improved herbicidal properties.

$R^5$ is selected from the group consisting of: allyl; substituted alkyl wherein the alkyl group is substituted with a substituent selected from the group consisting of alkoxy, alkylthio, optionally substituted phenyl, and optionally substituted heterocycle; or

$COXR^4$ and $R^5$ together with the carbon to which they are attached form a substituted or unsubstituted 5- or 6-membered heterocyclic ring. The spirocyclic compounds are preferred. The synthesis of the compounds, and herbicidal (including plant growth regulating) uses and compositions, are disclosed.

EP 0 219 343 A2

TITLE MODIFIED see front page 0219343

DUNLENA PTY. LIMITED 62/2018/02

HERBICIDES

This invention relates to organic compounds having herbicial properties and plant growth regulating properties, to processes for the preparation of such compounds, to intermediates useful in the preparation of such compounds and to herbicidal compositions and processes utilizing such compounds and to plant growth regulating compositions and process utilizing such compounds.

The use of certain cyclohexane-13-dione derivatives as grass herbicides is known in the art. Thus, for example, the compendium "Agricultural Chemicals - Book II Herbicides 1983-84 Revision" (W.T. Thomson Editor, Thomson Publications, California USA) describes the cyclohexane-1,3-dione derivatives known commercially as Alloxydim sodium (methyl-3-[1-(alloyloxyimino)butyl]-4-hydroxy-6,6dimethy-1-2-oxocyclohex-1-one) and Sethoxydim (2-[1(ethoxyimino)butyl-5-[2-ethylthio]propyl-3-hydroxy--2-cyclohexen-1-one) as selective post-emergent herbidies. Alloydim and Sethoxydim have been disclosed in Australian Patent No. 464 655 and Australian Patent Application No.35,314/78 respectively.

Esters and amides of the general formula (1) (X=0 or $NR^6$) have been claimed to show herbicidal activity (Eur. Pat. Appl. EP 126,713 (Chemical Abstracts, 1985 102, 112934)).

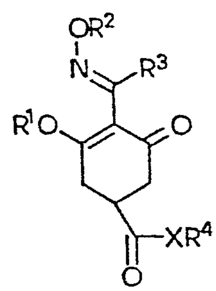

(1)

We have discovered that compounds similar to the general formula (1) but which bear an additional substituent at the 5-position exhibit particularly useful herbicidal activity with special selectivity for weed grasses in crops and plant growth regulating activity.

Accordingly, the invention provides a compound of the general formula (2)

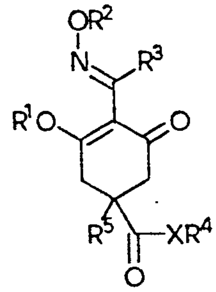

(2)

wherein $R^1$ is selected from the group consisting of: hydrogen; alkyl; alkenyl; alkynyl; substituted alkyl wherein the alkyl group is substituted with a substituent selected from the group consisting of alkoxy, alkylthio, optionally substituted phenyl, optionally substituted heterocycle; optionally substituted phenyl; optionally substituted heterocycle; alkyl sulfonyl; optionally substituted benzene sulfonyl; an acyl group; and an inorganic or organic cation;

$R^2$ is selected from the group consisting of: alkyl; alkenyl; haloalkenyl; alkynyl; haloalkynyl; substituted alkyl wherein the alkyl group is substituted with a substituent selected from the group consisting of halogen, alkoxy, alkylthio, optionally substituted phenyl, and optionally substituted heterocycle; optionally substituted phenyl; and optionally substituted heterocycle;

$R^3$ is selected from the group consisting of: alkyl; fluoroalkyl; alkenyl; alkynyl; and optionally substituted phenyl;

X = O or $NR^6$

$R^4$ is selected from the group consisting of: hydrogen; alkyl alkenyl; alkynyl; substituted alkyl wherein the alkyl group is substituted with a substituent selected from the group consisting of alkoxy, alkylthio, optionally substituted phenyl, and optionally substituted heterocycle; optionally substituted phenyl; and optionally substituted heterocycle;

$R^5$ is selected from the group consisting of: alkyl; substituted alkyl wherein the alkyl group is substituted with a substituent selected from the group consisting of alkoxy, alkylthio, optionally substituted phenyl, and optionally substituted heterocycle;

$R^6$ is selected from the group consisting of hydrogen; alkyl; substituted alkyl wherein the alkyl group is substituted with a substituent selected from the group consisting of alkoxy, alkylthio, optionally substituted phenyl, and optionally substituted heterocycle; optionally substituted phenyl; and optionally substituted heterocycle.

<u>OR</u>

$R^6$ and $R^4$ together with the nitrogen to which they are attached form a substituted or unsubstituted heterocyclic ring.

OR

$COXR^4$ and $R^5$ together with the carbon to which they are attached form a substituted or unsubstituted 5- or 6-membered heterocyclic ring. These spirocyclic compounds of the general formula (3) are a preferred group of compounds. For these novel structures $R^1$, $R^2$ , $R^3$ and X.

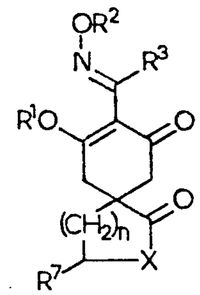

(3)

are as specified above while $R^7$ is selected from the group consisting of: hydrogen; alkyl; substituted alkyl wherein the alkyl group is substituted with a substituent selected from the group consisting of alkoxy, alkylthio, optionally substituted phenyl, and optionally substituted heterocycle; and n is 1 or 2.

It should be noted that derivatives of other spirocyclic cyclohexane-1,3-dione systems have been claimed as herbicides: Jpn. Kokai Tokkyo Koho JP 57,183,746 [82,183,746] (Chemical Abstracts, 1983, 98, 143011p); Jpn. Kokai Tokkyo Koho JP 58,144,384 [83,144,384] (Chemical Abstracts, 1984, 100, 34401e); Ger. Offen. DE 3,410,492 (Chemical Abstracts, 1986, 104, 83799g).

In all of the above heterocycle means a mono- or poly-cyclic heterocyclic ring structure that contains one or more heteroatoms and may or may not be aromatic. Suitable heteroatoms are nitrogen, oxygen, sulphur. The heterocyclic ring preferably has more than four atoms in

the ring. Some examples of suitable heterocycle groups are thiophenyl, benzofuranyl, furanyl, morpholino, and pyridyl.

Preferably in all of the above alkyl, alkenyl, alkynyl means lower alkyl, alkenyl or alkynyl. More preferably alkyl, alkoxy, alkylthio, haloalkyl, alkyl sulphonyl or substituted alkyl groups contain 1 to 6 carbon atoms and alkenyl, alkynyl, haloalkenyl, or haloalkynyl groups contain 2 to 6 carbon atoms.

It should be recognized that when $R_1$ is hydrogen the compounds of the invention may exist in any one of four tautomeric forms as shown below

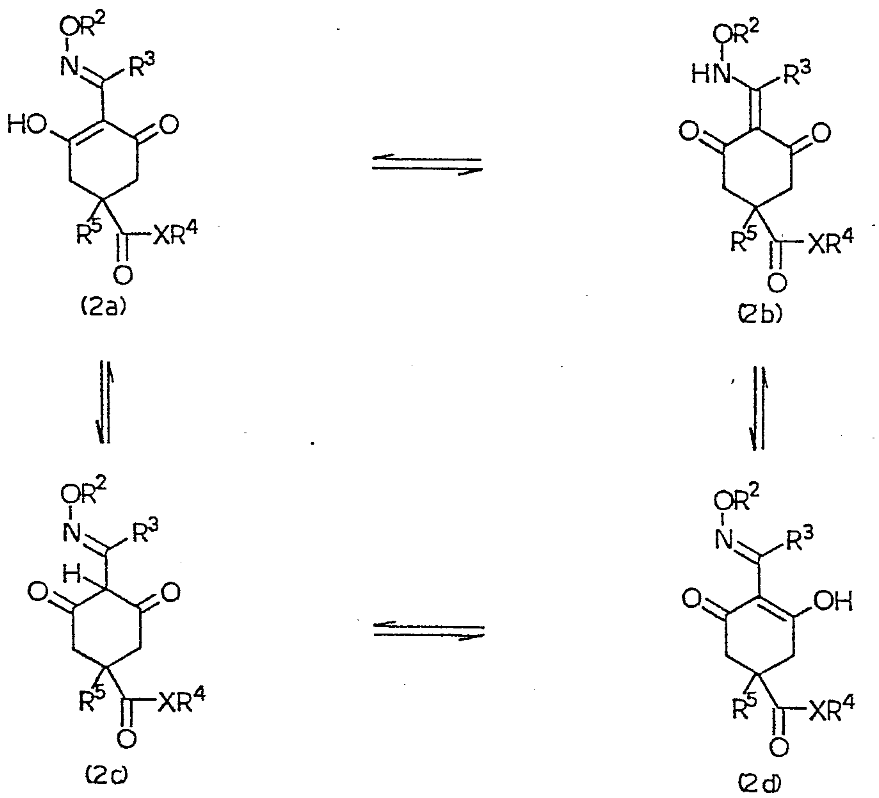

(2a) (2b) (2c) (2d)

Particularly preferred choices for $R^1$ include hydrogen and the alkali metals.

Particularly preferred choices for $R^2$ include ethyl and allyl.

Particularly preferred choices for $R^3$ include ethyl and n-propyl.

Particularly preferred choices for X and n for compounds of formula (3) are 0 and 1 respectively.

Specific examples of the compounds of the invention include those compounds detailed in Table 1 below. Some examples of compounds of formula (3) of the invention wherein X is 0 and n is 1 are detailed in Table 2 below.

Table 1

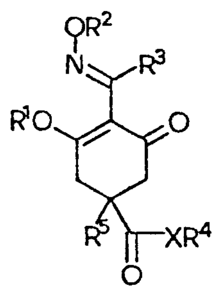

| Compound | X | $R^1$ | $R^2$ | $R^3$ | $R^4$ | $R^5$ |
|---|---|---|---|---|---|---|
| 2.1 | 0 | H | ethyl | n-propyl | methyl | methyl |
| 2.2 | 0 | H | ethyl | n-propyl | methyl | ethyl |
| 2.3 | 0 | H | ethyl | n-propyl | methyl | n-propyl |
| 2.4 | 0 | H | ethyl | n-propyl | methyl | isopropyl |
| 2.5 | 0 | H | ethyl | n-propyl | methyl | butyl |
| 2.6 | 0 | H | allyl | n-propyl | methyl | methyl |
| 2.7 | 0 | H | allyl | n-propyl | methyl | ethyl |
| 2.8 | 0 | H | allyl | n-propyl | methyl | n-propyl |
| 2.9 | 0 | H | allyl | n-propyl | methyl | isopropyl |
| 2.10 | 0 | H | allyl | n-propyl | methyl | butyl |
| 2.11 | 0 | H | ethyl | n-propyl | H | methyl |
| 2.12 | 0 | H | ethyl | n-propyl | H | ethyl |
| 2.13 | 0 | H | ethyl | n-propyl | H | n-propyl |
| 2.14 | $NCH_3$ | H | ethyl | n-propyl | methyl | ethyl |
| 2.15 | $NCH_3$ | H | allyl | n-propyl | methyl | ethyl |
| 2.16 | $NCH_3$ | H | ethyl | n-propyl | methyl | n-propyl |
| 2.17 | 0 | Na | ethyl | n-propyl | methyl | ethyl |
| 2.18 | NCH3 | Na | ethyl | n-propyl | methyl | ethyl |

Table 2

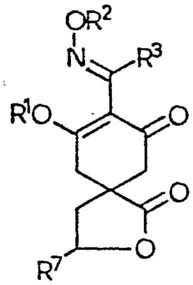

(3)

| Compound | $R^1$ | $R^2$ | $R^3$ | $R^7$ |
|---|---|---|---|---|
| 3.1 | H | ethyl | methyl | H |
| 3.2 | H | ethyl | ethyl | H |
| 3.3 | H | ethyl | n-propyl | H |
| 3.4 | Na | ethyl | n-propyl | H |
| 3.5 | H | cyclopentyl | n-propyl | H |
| 3.6 | H | allyl | methyl | H |
| 3.7 | H | allyl | n-propyl | H |
| 3.8 | H | 3-chloro-2-propenyl | n-propyl | H |
| 3.9 | H | ethyl | ethyl | methyl |
| 3.10 | H | ethyl | n-propyl | methyl |
| 3.11 | H | allyl | n-propyl | methyl |
| 3.12 | H | 3-chloro-2-propenyl | n-propyl | methyl |
| 3.13 | H | ethyl | n-propyl | ethyl |
| 3.14 | H | allyl | n-propyl | ethyl |
| 3.15 | H | 3-chloro-2-propenyl | n-propyl | ethyl |
| 3.16 | H | ethyl | n-propyl | n-propyl |
| 3.17 | H | allyl | n-propyl | n-propyl |
| 3.18 | H | 3-chloro-2-propenyl | n-propyl | n-propyl |
| 3.19 | H | ethyl | n-propyl | n-butyl |
| 3.20 | H | allyl | n-propyl | n-butyl |
| 3.21 | H | ethyl | n-propyl | n-pentyl |
| 3.22 | H | allyl | n-propyl | n-pentyl |
| 3.23 | H | ethyl | n-propyl | n-hexyl |
| 3.24 | H | allyl | n-propyl | n-hexyl |
| 3.25 | H | ethyl | n-propyl | isopropyl |
| 3.26 | H | ethyl | n-propyl | isobutyl |
| 3.27 | H | ethyl | n-propyl | $CH_3CH_2OCH_2$ |
| 3.28 | H | ethyl | n-propyl | $CH_3SCH_2$ |
| 3.29 | H | allyl | n-propyl | $CH_3SCH_2$ |
| 3.30 | H | ethyl | n-propyl | $CH_3CH_2SCH_2$ |

The compounds of this invention may be prepared from 3,5-dimethoxybenzoic acid by reduction with an alkali metal in liquid ammonia followed by addition of an appropriate electrophile (cf. Aust. J. Chem., 1981, 34, 675). (Scheme 1). The 1-substituted 3,5-dimethoxy-2,5-cyclohexadiene-1-carboxylic acid derivatives (4) thus obtained may be converted to the ester derivatives (6, X=O) and to the amide derivatives (6, X=$NR^6$) by standard literature methods. By way of non-limiting example, the esters (6, X=O) may be prepared by a two step method involving first reaction of (4) with an alcohol in the presence of an acid to afford the derivatives (5), followed by treatment of compounds (5) with aqueous acid (Scheme 1). The esters (6) may also be prepared by a three step method involving reduction of esters of of 3,5-dimethoxybenzoic acid with an alkali metal in liquid ammonia followed by addition of an electrophile followed by treatment with aqueous acid. Also, by way of non-limiting example, the amides (6, X=$NR^6$) may be prepared by a three step method involving first reaction of (4) with 1,1'-carbonyldiimidazole followed by treatment with an amine, other than a tertiary amine, to afford the derivatives (7), which upon treatment with aqueous acid give the amide derivatives (6, X=$NR^6$). These ester and amide derivatives may be acylated at the 4-position by a Fries rearrangement reaction to afford compounds (8), and the acylated derivatives (8) reacted with alkoxyamines to afford derivatives of the general formula (2) wherein $R^1$ is hydrogen. Hydrolysis of the esters (2, X=O) may provide further compounds of the invention of the general formula (2, X=O) wherein $R^4$ is hydrogen.

We have found that the use of 1,2-dihaloalkanes as electrophile in the reaction shown in Scheme 1 provides a particularly efficaceous method of making lactone derivatives of formula (3) wherein X is O and

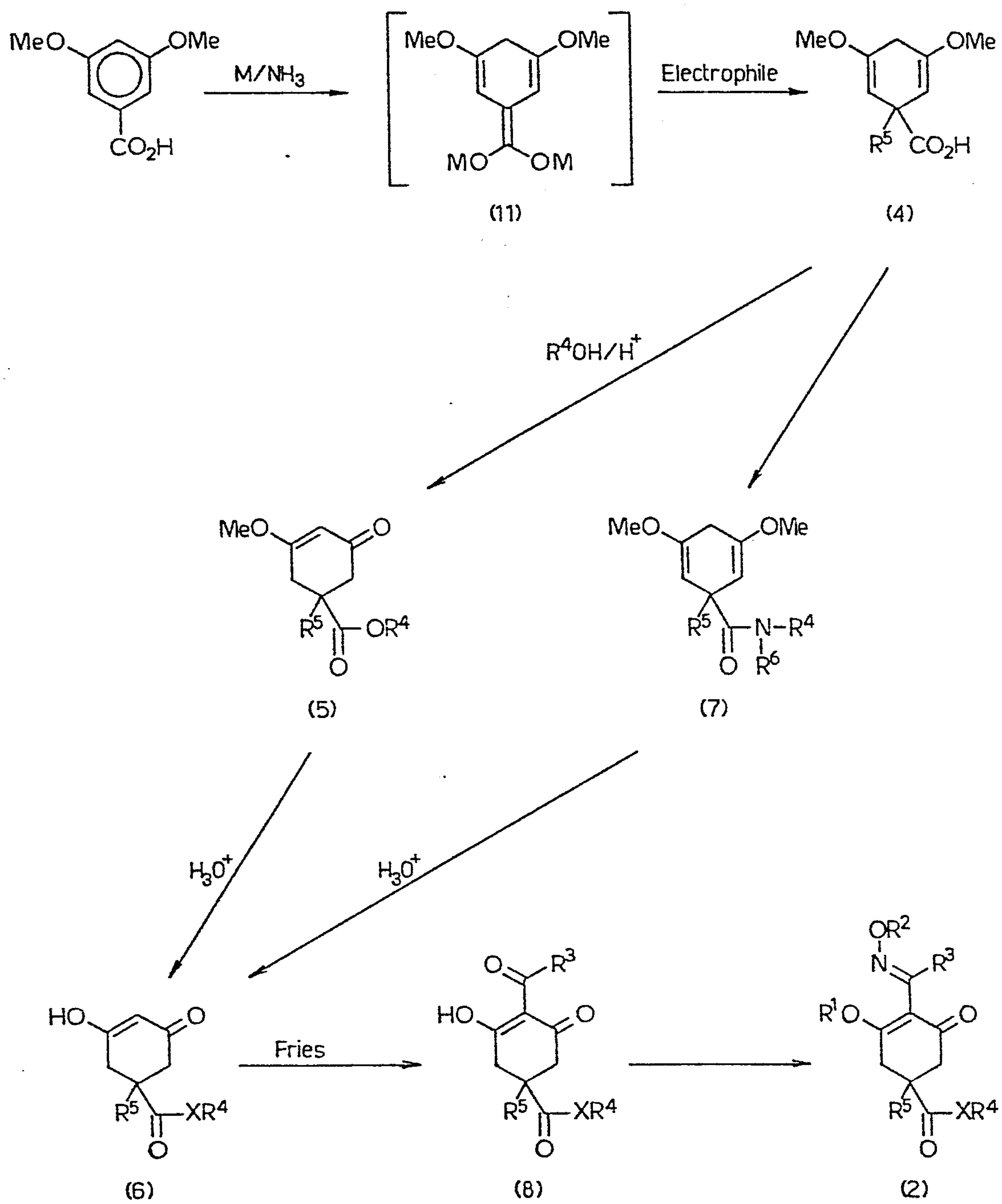


Scheme 1

n is 1 and accordingly we provide a novel process for the production of lactone derivatives of formula (3) wherein X is O and n is 1 wherein 3,5-dimethoxybenzoic acid is reduced by an alkali metal in liquid ammonia followed by the addition of an appropriately substituted 1,2-dihaloalkane to produce, after acid hydrolysis, 9-hydroxy-2-oxaspiro[4.5]dec-8-ene-1,7-dione derivatives (9).

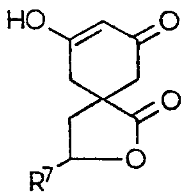

(9)

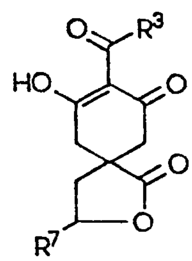

(10)

Compounds (9) may be acylated at the 8-position by a Fries rearrangement reaction to afford the compounds (10) and compounds (10) may be reacted with an appropriate alkoxyamine to produce compounds of formula (3).

We have also found that the use of oxirane derivatives as electrophile in place of 1,2-dihaloalkane derivatives in the reaction shown in Scheme 1 provides an efficaceous method of making lactone derivatives of formula (3) wherein X is O and n is 1.

Compounds of the invention of formula (2) wherein $R^1$ is an inorganic or organic cation may be prepared from compounds of the invention of formula (2) wherein $R^1$ is hydrogen by reacting these latter compounds with an inorganic or organic salt. For example, the compounds of formula (2) wherein $R^1$ is an alkali metal ion may be prepared by reacting the corresponding compounds (2) wherein $R^1$ is hydrogen with the appropriate alkali metal hydroxide or alkoxylate.

The compounds of formula (2) may be applied directly to the plant (post-emergence application) or to the soil before the emergence of the plant (pre-emergence application). However, the compounds are, in general, more effective when applied to the plant post-emergence.

The compounds of formula (2) may be used on their own to inhibit the growth of, severely damage, or kill plants but are preferably used in the form of a composition comprising a compound of the invention in admixture with a carrier comprising a solid or liquid diluent. Therefore, in yet a further aspect the invention provides plant growth inhibiting, plant damaging, or plant killing compositions comprising a compound of formula (2) as hereinbefore defined and an inert carrier therefor.

Certain of the compounds of formula (2) exhibit useful plant growth regulating activity. For example, while certain compounds of formula (2) show selective herbicidal activity against wild grasses in crops of cultivated plants, at some rates of application they exhibit plant growth regulating effects in said crops.

Plant growth regulating effects may be manifested in a number of ways. For example, suppression of apical dominance, stimulation of auxiliary bud growth, stimulation of early flowering and seed formation, enhancement of flowering and increase in seed yield, stem thickening, stem shortening and tillering. Plant growth regulating effects shown by compounds of the invention include, for example, tillering and stem shortening in crops such as wheat and barley.

Accordingly in a still further aspect the invention provides a process for regulating the growth of a plant which process comprises applying to the plant, to the seed of the plant, or to the growth medium of the plant, an effective amount of a compound of formula (2), as hereinbefore defined.

To effect the plant growth regulating process of the present invention the compounds of formula (2) may be applied directly to the plant (post-emergence application) or to the seed or soil before the emergence of the plant (pre-emergence) application.

The compounds of formula (2) may be used on their own to regulate the growth of plants but in general are preferably used in the form of a composition comprising a compound of the invention in admixture with a carrier comprising a solid or liquid diluent. Therefore, in a still further aspect the invention provides plant growth regulating compositions comprising a compound of formula (2) as hereinbefore defined and an inert carrier therefor.

The compositions of the present invention may be in the form of solids, liquids or pastes. The compositions include both dilute compositions which are ready for immediate use and concentrated compositions which may require dilution before use. Therefore, the concentration of the active ingredient in the compositions of the present invention will vary depending on the type of formulation and whether the composition is ready for use such as, for example, a dust formulation or an aqueous emulsion or whether the composition is a concentrate such as, for example, an emulsifiable concentrate or a wettable powder, which is suitable for dilution before use. The present invention includes both types of composition, accordingly the compositions to the present invention comprise from 1 ppm to 99% by weight of active ingredient.

The solid compositions may be in the form of powders, dusts, pellets, grains, and granules wherein the active ingredient is mixed with a solid diluent. Powders and dusts may be prepared by mixing or grinding the active ingredient with a solid carrier to give a finely divided composition. Granules, grains and pellets may be prepared by bonding the

active ingredient to a solid carrier, for example, by coating or impregnating the preformed granular solid carrier with the active ingredient or by agglomeration techniques.

Examples of solid carriers include: mineral earths and clays such as, for example, kaolin, bentonite, kieselguhr, Fuller's earth, Attaclay, diatomaceous earth, talc, chalk, dolomite, limestone, lime, calcium carbonate, powdered magnesia, magnesium oxide, magnesium sulfate, gypsum, calcium sulfate, pyrophyllite, silicic acid, silicates and silica gels; fertilizers such as, for example, ammonium sulfate, ammonium phosphate, ammonium nitrate and urea; natural products of vegetable origin such as, for example, grain meals and flours, bark meals, wood meals, nutshell meals and cellulosic powders; and synthetic polymeric materials such as, for example, ground or powdered plastics and resins.

Alternatively, the solid compositions may be in the form of dispersible or wettable dusts, powders, granules or grains wherein the active ingredient and the solid carrier are combined with one or more surface active agents which act as wetting, emulsifying and/or dispersing agents to facilitate the dispersion of the active ingredient in liquid.

Examples of surface active agents include those of the cationic, anionic and non-ionic type. Cationic surface active agents include quaternary ammonium compounds, for example, the long chain alkylammonium salts such as cetyltrimethylammonium bromide. Anionic surface active agents include: soaps or the alkali metal, alkaline earth metal and ammonium salts of fatty acids; the alkali metal, alkaline earth metal and ammonium salts of ligninsulfonic acid; the alkali metal, alkaline earth metal and ammonium salts of arylsulfonic acids including the salts of naphthalenesulfonic acids such as butylnaphthalenesulfonic acid, the di- and tri- isopropylnaphthalenesulfonic acids, the salts of the

condensation products of sulfonated naphthalene and naphthalene derivatives with formaldehyde, the salts of the condensation products of sulfonated naphthalene and naphthalene derivatives with phenol and formaldehyde, and the salts of alkylarylbenzenesulfonic acids such as dodecylbenzenesulfonic acid; the alkali metal, alkaline earth metal and ammonium salts of the long chain mono esters of sulfuric acid or alkylsulfates such as laurylsulfate and the mono esters of sulfuric acid with fatty alcohol glycol ethers. Nonionic surface active agents include: the condensation products of ethylene oxide with fatty alcohols such as oleyl alcohol and cetyl alcohol; the condensation products of ethylene oxide with phenols and alkylphenols such as isooctylphenol, octylphenol and nonylphenol; the condensation products of ethylene oxide with castor oil; the partial esters derived from long chain fatty acids and hexitol anhydrides, for example sorbitan monolaurate, and their condensation products with ethylene oxide; ethylene oxide/propylene oxide block copolymers; lauryl alcohol polyglycol ether acetal; and the lecithins.

The liquid compositions may comprise a solution or dispersion of the active ingredient in a liquid carrier optionally containing one or more surface active agents which act as wetting, emulsifying and/or dispersing agents. Examples of liquid carriers include: water; mineral oil fractions such as, for example, kerosene, solvent naphtha, petroleum, coal tar oils and aromatic hydrocarbons such as, for example, paraffin, cyclohexane, toluene, the xylenes, tetrahydronaphthalene and alkylated naphthalenes; alcohols such as, for example, methanol, ethanol, propanol, isopropanol, butanol, cyclohexanol and propylene glycol; ketones such as, for example, cyclohexanone and isophorone; and strongly polar organic

solvents such as, for example, dimethylformamide, dimethylsulfoxide, N-methylpyrrolidone and sulfolane.

A preferred liquid composition comprises an aqueous suspension, dispersion or emulsion of the active ingredient which is suitable for application by spraying, atomizing or watering. Such aqueous compositions are generally prepared by mixing concentrated compositions with water. Suitable concentrated compositions include emulsion concentrates, pastes, oil dispersions, aqueous suspensions and wettable powders. The concentrates are usually required to withstand storage for prolonged periods and after such storage to be capable of dilution with water to form aqueous preparations which remain homogeneous for a sufficient time to enable them to be applied by conventional spray equipment. The concentrates conveniently contain from 20 to 99%, preferably 20 to 60%, by weight of active ingredient.

Emulsion or emulsifiable concentrates are conveniently prepared by dissolving the active ingredient in an organic solvent containing one or more surface active agents used in the formulation and the salts generated in situ by the use of the appropriate organic or inorganic base.

The mode of application of the compositions of the invention will depend to a large extent on the type of composition used and the facilities available for its application. Solid compositions may be applied by dusting or any other suitable means for broadcasting or spreading the solid. Liquid compositions may be applied by spraying, atomizing, watering, introduction into the irrigation water, or any other suitable means for broadcasting or spreading the liquid.

The rate of application of the compounds of the invention will depend on a number of factors including, for example, the compound chosen

for use, the identity of the plants whose growth is to be inhibited, the formulations selected for use and whether the compound is to be applied for foliage or root uptake. As a general guide, however, an application rate of from 0.005 to 20 kilograms per hectare is suitable while from 0.01 to 5.0 kilograms per hectare may be preferred.

The compositions of the invention may comprise, in addition to one or more compounds of the invention, one or more compounds not of the invention but which possess biological activity. For example, as herein before indicated the compounds of the invention are in general substantially more effective against monocotyledonous plants or grass species than against dicotyledonous plants or broad-leaved species. As a result, in certain applications the herbicidal use of the compounds of the invention alone may not be sufficient to protect a crop. Accordingly in yet a still further embodiment the invention provides a herbicidal composition comprising a mixture of at least one herbicidal compound of formula (2) as hereinbefore defined with at least one other herbicide.

The invention is now illustrated by but in no way limited to the following examples

EXAMPLE 1

Preparation of Methyl 4-[1-(Ethoxyimino)butyl]-1-ethyl-3-hydroxy-5-oxo-3-cyclohexene-1-carboxylate (2.2)

(a) 1-Ethyl-3,5-dimethoxy-2,5-cyclohexadiene-1-carboxylic acid (4.1)

To a stirred solution of 3,5-dimethoxybenzoic acid (20.00 g, 109.8 mmol) in dry tetrahydrofuran (200 ml) and anhydrous ammonia (c. 300 ml) under argon and cooled to -78° was added portionwise lithium metal (c. 1.9 g, 274 mmol) until a deep blue colour persisted. Ethyl bromide (20.5 ml, 274 mmol) was added in one portion and the solution stirred for 1 h at -78°. The ammonia was allowed to evaporate and the tetrahydrofuran was evaporated under a stream of argon. To the residue was added water (100 ml) followed by chloroform (200 ml). The stirred mixture was cooled to 0° and the aqueous layer was acidified to pH 3-4 by the careful addition of 12M hydrochloric acid (c. 30 ml). The mixture was transferred to a separating funnel and the chloroform layer collected. The aqueous layer was further extracted with chloroform (200 ml then 50 ml), the combined organic extracts dried (magnesium sulfate) and evaporated. The residue was crystallized from dichloromethane/light petroleum (b.p. 40-60°), after being chromatographed on silica gel (c. 10 g) using chloroform as the eluant, to afford the title compound (4.1) (17.11 g, 73%), m.p. 141-144° (Found C, 62.3; H, 7.3. $C_{11}H_{16}O_4$ requires C, 62.2; H, 7.6%). $^1$H n.m.r. δ ($CDCl_3$) 8.50, broad s, OH; 4.65, s, H2 and H6; 3.62, s, 2x$OCH_3$; 2.77, s, H4; 1.76, q, J 7 Hz, $\underline{CH_2}CH_3$; 0.83, t, J 7 Hz, $CH_2\underline{CH_3}$. Mass spectrum (C.I. (Chemical Ionization)): m/z 213 $(M+1)^{+\cdot}$. $\nu_{max}$ (Infrared) (KBr): 3400 (broad, OH), 2600 (broad, OH), 1695, (s, CO), 1680 (s, CO), 1655 (m, C=C) $cm^{-1}$.

(b) Methyl 1-Ethyl-3-methoxy-5-oxo-3-cyclohexene-1-carboxylate (5.1)

To a stirred solution of methanol (100 ml) and conc. sulfuric acid (5 ml) was added a solution of the acid (4.1) (10.0 g, 47.1 mmol) in methanol (100 ml). The mixture was refluxed for 2 days and then the methanol evaporated. Chloroform (100 ml) was added to the residue and the mixture neutralized by the addition of saturated sodium hydrogen carbonate. The chloroform layer was collected, dried (magnesium sulfate) and evaporated to afford the ester (5.1) (8.38 g, 84%) as a yellow oil. $^1H$ n.m.r. $\delta$ ($CDCl_3$) 5.31, s, H4: 3.71, s, $OCH_3$; 3.68, s, $OCH_3$; 3.07-2.70, m, H2 or H6; 2.57-2.12, m, H2 or H6; 1.74, q, J 7 Hz, $\underline{CH_2}CH_3$; 0.88, t, J 7 Hz, $CH_2\underline{CH_3}$. Mass spectrum (C.I.): m/z 213 $(M+1)^{+\cdot}$. $\nu_{max}$ (thin film): 1730 (s, 1-CO), 1655 (s), 1610 (s) $cm^{-1}$.

(c) Methyl 1-Ethyl-3-hydroxy-5-oxo-3-cyclohexene-1-carboxylate (6.1)

To a stirred solution of the ester (5.1) (8.38 g, 39.5 mmol) and in tetrahydrofuran (80 ml) was added water (9 ml) followed by 12M hydrochloric acid (1 ml). The mixture was left at room temperature overnight. The tetrahydrofuran was evaporated and chloroform (150 ml) was added to the residue. The chloroform layer was collected, washed with brine (40 ml), dried (magnesium sulfate) and evaporated. The residue was recrystallized from acetone/diethyl ether to afford the title compound (6.1) (6.75 g, 86%) as colourless crystals, m.p. 101-103° (Found C, 60.9; H, 7.3. $C_{10}H_{14}O_4$ requires C, 60.6; H, 7.1%). $^1H$ n.m.r. $\delta$ ($CDCl_3$) 10.66, s, OH; 5.42, s, H4; 3.66, s, $OCH_3$; 2.90, 2.36, AB q, J 17 Hz, H2 and H6; 1.72, q, J 7 Hz, $\underline{CH_2}CH_3$; 0.85, t, J 7 Hz, $CH_2\underline{CH_3}$. Mass spectrum (C.I.): m/z 199 $(M+1)^{+\cdot}$. $\nu_{max}$ (KBr): 3450 (broad, OH), 2560 (broad, OH), 1735 (s, CO), 1620 (s) $cm^{-1}$.

(d) Methyl 4-Butyryl-1-ethyl-3-hydroxy-5-oxo-3-cyclohexene-1-carboxylate (8.1)

A stirred solution of the ester (6.1) (4.217 g, 21.27 mmol) and dimethylaminopyridine (524 mg, 4.29 mmol) in triethylamine (8.9 ml, 63.9 mmol) and butyric anhydride (10.44 ml, 63.9 mmol) under argon was heated to 80° for 1 h. After this time methanol (5 ml) was added and the mixture heated a further 5 min. The mixture was cooled and dissolved in a mixture of diethyl ether (150 ml) and 3M hydrochloric acid (40 ml). The ether layer was collected, and the aqueous phase further extracted with ether (50 ml). The combined ether extracts were washed with brine (40 ml), dried (magnesium sulfate) and evaporated. The excess butyric acid was removed by distillation (2 Torr, pot temperature 80°) and the pot residue chromatographed on silica using chloroform/50% light petroleum (b.p. 40-60°) as the eluant to afford (8.1) (2.535 g, 44%) as a pale yellow oil. $^1$H n.m.r. δ ($CDCl_3$) 18.12, s, OH; 3.68, s, $OCH_3$; 3.28-2.27, m, H2 and H6; 2.97, t, J 7 Hz, 4-$COCH_2$; 1.90-1.40, m, 4H; 0.97, t, J 7 Hz, $CH_3$; 0.88, t, J 7 Hz, $CH_3$. Mass spectrum (E.I.): m/z 268 ($M^{+\cdot}$, accurate mass 268.131. $C_{14}H_{20}O_5$ requires 268.131). $\nu_{max}$ (thin film): 1730 (s, CO), 1665 (s, CO) $cm^{-1}$.

Methyl 4-((1-Ethoxyimino)butyl)-1-ethyl-3-hydroxy-5-oxo-3-cyclohexene-1-carboxylate (2.2)

A solution of (8.1) (1.300 g, 4.84 mmol) and ethoxyamine hydrochloride (945 mg, 9.69 mmol) in ethanol (30 ml) and pyridine (2 ml) was stirred at room temperature for 24 h. The solvents were evaporated and diethyl ether (200 ml) was added to the residue followed by 1M hydrochloric acid (40 ml). The ether layer was collected, washed with brine (20 ml) and dried (magnesium sulfate). The ether extract was

evaporated and the residue subjected to radial chromatography (4 mm thickness silica disc) using dichloromethane/40% light petroleum (b.p. 40-60°) as the eluant to afford the title compound (2.2) (1.097 g, 73%) as a colourless oil. $^1$H n.m.r. δ ($CDCl_3$) 14.8, broad s, OH; 4.08, q, J 7 Hz, $OCH_2CH_3$; 3.68, s, $OCH_3$; 2.98, 2.41, AB q, J 18 Hz, H2 and H6; 2.91, t, J 6 Hz, $N{=}CCH_2$; 1.70, q, J 7 Hz, 1-$CH_2CH_3$; 1.72-1.30, m, 2H; 1.30, t, J 7 Hz, $OCH_2CH_3$; 0.91, t, J 7 Hz, $CH_3$; 0.86, t, J 7 Hz, $CH_3$. Mass spectrum (C.I.): m/z 312 ($(M+1)^{+\cdot}$, accurate mass 312.179. $C_{16}H_{26}NO_5$ requires 312.181). $\nu_{max}$ (thin film): 1730 (s, CO), 1660 (s), 1605 (s) $cm^{-1}$.

EXAMPLE 2

Preparation of Methyl 4-((1-Allyloxyimino)butyl)-1-ethyl-3-hydroxy-5-oxo-3-cyclohexene-1-carboxylate (2.7)

A solution of (8.1) (1.585 g, 5.91 mmol) and allyloxyamine hydrochloride (1.309 g, 12.6 mmol) in ethanol (20 ml) and pyridine (3 ml) was stirred at room temperature overnight. The reaction was worked up exactly as described for the preparation of compound (2.2) to afford the title compound (2.7) (1.185 g, 62%) as a pale yellow oil. $^1$H n.m.r. δ ($CDCl_3$) 14.40, s, OH; 6.20-5.74, m, 1H; 5.50-5.18, m, 2H; 4.51, d, J 6 Hz, $N{-}O{-}CH_2$; 3.60, s, $OCH_3$; 3.23-2.70, m, 4H; 2.67-2.15, m, 2H; 1.70, q, J 6 Hz, C1-$CH_2CH_3$; 1.72-1.25, m, 2H; 0.93, t, J 6 Hz, $CH_3$; 0.89, t, J 6 Hz, $CH_3$. Mass spectrum (C.I.): m/z 323 $(M+1)^{+\cdot}$. $\nu_{max}$ (thin film): 1730 (s, CO), 1655 (s, CO), 1600 (s) $cm^{-1}$.

EXAMPLE 3

Preparation of 4-((1-Ethoxyimino)butyl)-1-ethyl-3-hydroxy-5-oxo-3-cyclohexene-1-carboxylic Acid (2.11)

The ester (2.2) (277 mg, 0.889 mmol) was added to a solution of sodium hydroxide (200 mg) in water (20 ml). The mixture was heated to 80° under argon for 1 h. The cooled solution was acidified to pH 1 by the addition of 12M hydrochloric acid and extracted with chloroform (2x50 ml). The dried (magnesium sulfate) chloroform extracts were evaporated to afford after crystallization from diethyl ether/light petroleum (b.p. 40-60°) the title compound (2.11) (232 mg, 88%) as a colourless crystals, m.p. 98-99° (Found C, 60.7; H, 7.8; N, 4.5. $C_{15}H_{23}NO_5$ requires C, 60.6; H, 7.8; N, 4.7%). $^1H$ n.m.r. $\delta$ ($CDCl_3$) 12.20, broad s, 2xOH; 4.10, q, J 7 Hz, $CH_3\underline{CH_2}O$; 3.20-2.30, m, 6H; 1.95-1.30, m, 4H; 1.31, t, J 7 Hz, $\underline{CH_3}CH_2O$; 0.93, t, J 6 Hz, $2xCH_3$. Mass spectrum (C.I.): m/z 298 $(M+1)^{+\cdot}$. $\nu_{max}$ (KBr): 3100 (broad, OH), 1725 (s, CO), 1600 (s) $cm^{-1}$.

EXAMPLE 4

Preparation of N,N-Dimethyl-4-(1-(ethoxyimino)butyl)-1-ethyl-3-hydroxy-5-oxo-3-cyclohexene-1-carboxamide (2.14)

(a) N,N-Dimethyl-1-ethyl-3,5-dimethoxy-2,5-cyclohexadiene-1-carboxamide (7.1)

To a stirred solution of compound (4.1) (5.027 g, 23.68 mmol) in 1,2-dichloroethane (50 ml) under argon was added 1,1'-carbonyldiimidazole (4.283 g, 26.41 mmol). The solution was stirred at room temperature for 1 h and then cooled to 0°. Excess dimethylamine (c. 6.0 g) was added and

the solution left at 0° for 48 h. The solvents were evaporated and the residue was dissolved in ethyl acetate (200 ml), and washed in turn with water (10 ml), brine (10 ml), 3N hydrochloric acid (20 ml) and brine (10 ml). The organic extract was dried (magnesium sulfate) and evaporated to afford the title compound (7.1) (5.305 g, 94%) as a colourless solid. Recrystallization from diethyl ether/light petroleum (b.p. 40-60°) gave (7.1) as colourless prisms, m.p. 79-80°. $^1H$ n.m.r. δ ($CDCl_3$) 4.46, s, H2 and H6; 3.60, s, $2xOCH_3$; 2.95, s, $N(CH_3)_2$; 2.77, d, J 3 Hz, H4; 1.82, q, J 6 Hz, $\underline{CH_2}CH_3$; 0.72, t, J 6 Hz, $CH_2\underline{CH_3}$. Mass spectrum (C.I.): m/z 240 $(M+1)^{+\cdot}$. $\nu_{max}$ (KBr): 1685 (s), 1620 (s) $cm^{-1}$.

(b) <u>N,N-Dimethyl-1-ethyl-3-hydroxy-5-oxo-3-cyclohexene-1-carboxamide (6.2)</u>

To a stirred solution of (7.1) (5.305 g, 22.16 mmol) in tetrahydrofuran (100 ml) was added 5M hydrochloric acid (10 ml). The solution was left overnight at room temperature. The tetrahydrofuran was evaporated, and to the aqueous solution was added chloroform (150 ml). The aqueous layer was neutralized by the addition of saturated sodium hydrogen carbonate solution and then acidified to pH 1-2 by the addition of 3M hydrochloric acid. The chloroform layer was removed, washed with brine (20 ml), dried (magnesium sulfate) and evaporated to afford (6.2) (4.511 g, 96%) as a colourless gum. $^1H$ n.m.r. δ ($CDCl_3$) 9.50, s, OH; 5.42, s, H4; 3.07, s, $N(CH_3)_2$; 3.02, 2.43, AB q, J 17 Hz, H2 and H6; 1.86, q, J 7 Hz, $\underline{CH_2}CH_3$; 0.90, t, J 7 Hz, $CH_2\underline{CH_3}$. Mass spectrum (C.I.): m/z 212 $(M+1)^{+\cdot}$ $\nu_{max}$ (thin film): 3600-2400 (complex broad bands, OH), 1615 (broad, s) $cm^{-1}$.

(c) N,N-Dimethyl-4-butyryl-1-ethyl-3-hydroxy-5-oxo-3-cyclohexene-1-carboxamide (8.2)

A stirred solution of the amide (6.2) (4.511 g, 21.35 mmol) and dimethylaminopyridine (440 mg, 3.60 mmol) in butyric anhydride (5.24 ml, 32.0 mmol) and triethylamine (4.46 ml, 32.0 mmol) was heated to 80° for 1 h under argon. Methanol (5 ml) was then added via the condensor and the reaction mixture heated for a further 5 min. Work-up exactly as described for the preparation of compound (8.1) afforded the title compound (8.2) (2.10 g, 35%) as a colourless solid, m.p. 47-47°. $^1$H n.m.r. δ ($CDCl_3$) 18.23, s, OH; 3.48-2.40, m, 8H; 3.06, s, $N(CH_3)_2$; 2.10-1.40, m, 4H; 0.97, t, J 7 Hz, $CH_3$; 0.93, t, J 7 Hz, $CH_3$. Mass spectrum (C.I.): m/z 282 $(M+1)^{+\cdot}$. $\nu_{max}$ (film): 3500 (broad, w, OH), 1665, (s, CO), 1625 (s, CO), 1565 (broad, s) $cm^{-1}$.

(d) N,N-Dimethyl-4-(1-(ethoxyimino)butyl)-1-ethyl-3-hydroxy-5-oxo-3-cyclohexene-1-carboxamide (2.14)

To a stirred solution of (8.2) (1.007 g, 3.58 mmol) in ethanol (20 ml) and pyridine (3 ml) was added ethoxyamine hydrochloride (563 mg, 5.78 mmol). The mixture was allowed to stand overnight and then worked up exactly as described for the preparation of (2.2) to afford crude (2.14) which was subjected to radial chromatography (4 mm thickness silica disc) using dichloromethane then dichloromethane/1% methanol as the eluant to give pure (2.14) (852 mg, 73%) as a colourless oil. $^1$H n.m.r. δ ($CDCl_3$) 14.97, s, OH; 4.02, q, J 6 Hz, $CH_3\underline{CH_2}O$; 3.30-2.35, m, 6H; 3.02, s, $N(CH_3)_2$; 1.95-1.20, m, 4H; 1.30, t, J 6 Hz, $\underline{CH_3}CH_2O$; 0.92, t, J 7 Hz, $CH_3$; 0.88, t, J 7 Hz, $CH_3$. Mass spectrum (C.I.): m/z 325 $(M+1)^{+\cdot}$. $\nu_{max}$ (thin film): 1650 (s), 1625 (s) $cm^{-1}$.

EXAMPLE 5

8-((1-Ethoxyimino)butyl)-9-hydroxy-2-oxaspiro[4.5]dec-8-ene-1,7-dione (3.3)

(a) Preparation of 9-Hydroxy-2-oxaspiro[4.5]dec-8-ene-1,7-dione (9.1)

Method 1

To a stirred solution of 3,5-dimethoxybenzoic acid (5.10 g, 28.0 mmol) in dry tetrahydrofuran (100 ml) and liquid ammonia (c. 200 ml) under argon and cooled to -78° was added portionwise lithium metal (c. 423 mg, c. 61.0 mmol) until a deep blue colour persisted. 1,2-Dibromoethane (8.42 g, 44.8 mmol) was added in one portion and the solution stirred for 1 h at -78°. The ammonia was allowed to evaporate and the solution was then stirred for 1 h at room temperature. The tetrahydrofuran was evaporated under a stream of nitrogen and to the residue was added water (50 ml) and chloroform (50 ml). The aqueous layer was carefully acidified to pH 1 by the addition of 3M hydrochloric acid and the chloroform layer collected. The aqueous solution was further extracted with chloroform (2x50 ml) and the combined organic extracts dried and evaporated. To the residue (5.67 g) was added tetrahydrofuran (50 ml) followed by 1.2 M hydrochloric acid (10 ml). The solution was stirred overnight at room temperature and then the tetrahydrofuran evaporated. The aqueous solution was cooled to 0° and the crystals which formed were collected by filtration and washed with ice-cold water (10 ml) to afford, after drying *in vacuo* at 50°, the title compound (9.1) (2.61 g, 51%), m.p. 162-164° (after recrystallization from acetone). [Reference to this compound has been made in the literature: J. Chem. Soc. Perkin Trans. 1, 1982, 1485; the literature method refers

to the use of the tetrahydropyranyl ether of 2-bromoethanol as the electrophile in the reductive alkylation reaction in place of 1-bromo-2-chloroethane]. $^{1}H$ n.m.r. δ ($CDCl_3$) 7.10, very broad s, OH; 5.50, s, H8; 4.35, t, J 6 HZ, H3; 2.82, 2.36, AB q, J 18 Hz, H6 and H10; 2.30, t, J 6 Hz, H4. The aqueous filtrate was extracted with ethyl acetate (2x50 ml). The organic extracts were washed with brine (25 ml), dried and evaporated. The residue (1.46 g) was subjected to radial chromatography using chloroform then chloroform/2.5% methanol as the eluant to afford 3,5-dimethoxybenzoic acid (101 mg, 2%) followed by the spirolactone (9.1) (582 mg, 11%).

Method 2

A stirred solution of 3,5-dimethoxybenzoic acid (25.0 g, 0.137 mol) in dry tetrahydrofuran (100 ml) and ammonia (c. 500ml) was reduced with lithium metal (c. 2.10 g, 0.303 mol) and treated with 1-bromo-2-chloroethane (32.2 g, 0.238 mol) as described above. To the residue obtained after removal of the solvents was added water (50 ml) followed by chloroform (200 ml). The aqueous layer was carefully acidified to pH 1-2 by the addition of 12 M hydrochloric acid. The chloroform layer was removed and the aqueous layer further extracted with chloroform (2x100 ml). The residue obtained from evaporation of the dried organic extracts was stirred with tetrahydrofuran (100 ml) and 2M hydrochloric acid (24 ml) overnight. The tetrahydrofuran was evaporated and the crystals which formed on cooling the aqueous solution were filtered off to afford (9.1) (20.4 g, 82%).

Method 3

The dianion (11, M=Li) was prepared from 3,5-dimethoxybenzoic acid (3) (5.00 g, 27.5 mmol) exactly as described in method 1. The ammonia was carefully removed (ice-bath) under a stream of argon. The resulting orange coloured solution was treated with oxirane gas (c. 9.0 g, c. 204 mmol) at 0°. The almost colourless solution was then evaporated under a stream of dry nitrogen and the residue treated as described in method 1 to afford (9.1) (2.16 g, 43%). The residue from the ethyl acetate extract was recrystallized to afford more (9.1) (580 mg, 12%).

(b) 8-Butyryl-9-hydroxy-2-oxaspiro[4.5]dec-8-ene-1,7-dione (10.1)

A stirred solution of the lactone (9.1) (2.020 g, 11.09 mmol) and 4-dimethylaminopyridine (207 mg, 1.69 mmol) in butyric anhydride (5.44 ml, 33.3 mmol) and triethylamine (4.64 ml, 33.3 mmol) was heated to 80° for 1 h under argon. To the cooled solution was added dichloromethane (10 ml) followed by diethyl ether (150 ml), and 3M hydrochloric acid (40 ml). The ether layer was separated, washed with brine (40 ml), dried and the ether evaporated. The excess butyric acid was removed by distillation (2 Torr, pot temperature 80°) and the pot residue chromatographed on silica using light petroleum (b.p. 40-60°)/25% dichloromethane as the eluant to afford the title compound (10.1) (1.796 g, 64%) as a pale cream solid. Recrystallization from diethyl ether/light petroleum (b.p. 40-60°) gave (10.1) as colourless prisms, m.p. 81-82° (Found: C, 62.2; H, 6.1. $C_{13}H_{16}O_5$ requires C, 61.9; H, 6.4%). Mass spectrum (C.I.): m/z 253 $(M+1)^{+\cdot}$. $^1H$ n.m.r. δ ($CDCl_3$) 18.24, s, OH; 4.37, t, J 6 Hz, H3; 3.30-2.20, m, 6H; 2.26, t, J 6 Hz, H4; 1.90-1.46, m, $\underline{CH_2}CH_3$; 1.00, t, J 5 Hz, $CH_3$. $\nu_{max}$ (film): 1760 (s, C=O), 1665 (s, C=O) $cm^{-1}$.

8-(1-(Ethoxyimino)butyl)-9-hydroxy-2-oxaspiro[4.5]dec-8-ene-1,7-dione (3.3)

A solution of (10.1) (891 mg, 3.53 mmol) and ethoxyamine hydrochloride (688 mg, 7.06 mmol) in ethanol (20 ml) and pyridine (2 ml) was stirred at room temperature for 48 h. The solvents were evaporated and diethyl ether (100 ml) was added to the residue followed by 1M hydrochloric acid (40 ml). The ether layer was separated, washed with brine (20 ml) and dried. The ether extract was evaporated and the residue (c. 1.0 g) subjected to radial chromatography using dichloromethane/1% methanol as the eluant to afford the title compound (3.3) (635 mg, 61%) as a pale yellow oil. Crystallization from diethyl ether/light petroleum (b.p. 40-60°) gave (3.3) as colourless needles, m.p. 79-80° (Found: C, 60.8; H, 7.2; N, 5.0. $C_{15}H_{21}NO_5$ requires C, 61.0; H, 7.2; N, 4.7%). Mass spectrum (C.I.): m/z 296 $(M+1)^{+\cdot}$. $^1H$ n.m.r. δ ($CDCl_3$) 15.32, broad s, OH; 4.33, t J 6 Hz, H3; 4.13, q, J 5 Hz, $OC\underline{H_2}CH_3$; 2.98, partly obscured t, J 6 Hz, $N{=}CCH_2$; 2.98, 2.49, AB q, J 18 Hz, H6 and H10; 2.28, t, J 6 Hz, H4; 1.75-1.20, m, $C\underline{H_2}CH_3$; 1.34, t, J 6 Hz, $OCH_2C\underline{H_3}$; 1.00, t, J 6 Hz, $CH_3$. $\nu_{max}$ (film): 1760 (s, C=O), 1665 (s, C=O) $cm^{-1}$.

EXAMPLE 6

Preparation of Compound (3.4), the Sodium Salt of Compound (3.3)

To a stirred solution of (3.3) (353 mg, 1.20 mmol) in tetrahydrofuran (10 ml) was added 0.133 M sodium hydroxide (9.04 ml). The solution was evaporated to dryness and titurated with chloroform to afford compound (3.4), the sodium salt of the lactone (3.3), (289 mg, 76%), decomposes at 198-206°.

EXAMPLE 7

Preparation of 8-((1-Allyloxyimino)butyl)-9-hydroxy-2-oxaspiro[4.5]dec-8-ene-1,7-dione (3.7)

A soluton of (10.1) (1.187 g, 4.71 mmol) and allyloxyamine hydrochloride (1.024 g, 9.35 mmol) in ethanol (20 ml) and pyridine (3 ml) was stirred at room temperature overnight. The reaction was worked up exactly as described for the preparation of compound (3.3) to afford (3.7) (1.25 g, 87%) as a pale yellow oil. Crystallization from diethyl ether/light petroleum (b.p. 40-60°) gave compound (3.7) as colourless crystals m.p. 47-48°. $^1$H n.m.r. δ ($CDCl_3$) 14.34, s, OH; 6.22-5.73, m, 1H; 5.57-5.20, m, 2H; 4.53, d, J 6 Hz, N-O-$CH_2$; 4.32, t, J 7 Hz, H3; 2.97, partly obscured t, J 6 Hz, N=$CCH_2$; 2.95, 2.47, AB q, J 16 Hz, H6 and H10; 2.25, t, J 7 Hz, H4; 1.80-1.30, m, 2H; 0.98, t, J 6 Hz, $CH_3$. Mass spectrum (C.I.): m/z 308 $(M+1)^{+\cdot}$. $\nu_{max}$ (thin film): 1765 (s, CO), 1655 (s, CO), 1600 (s) $cm^{-1}$.

EXAMPLE 8

8-((1-Ethoxyimino)butyl)-3-(ethylthiomethyl)-9-hydroxy-2-oxaspiro[4.5]dec-8-ene-1,7-dione (3.30)

(a) 3-(Ethylthiomethyl)-9-hydroxy-2-oxaspiro[4.5]dec-8-ene-1,7-dione (9.2)

To a solution of the dianion (11, M=Li) prepared from 3,5-dimethoxybenzoic acid (5.50 g, 30.2 mmol), in tetrahydrofuran (100 ml) and ammonia (c. 200 ml) under argon at -78° was added (ethylthiomethyl)oxirane (J. Am. Chem. Soc., 1950, 72, 4000) (4.62 g, 39.1 mmol). The reaction mixture was stirred at -78° for 1 h. The

ammonia was allowed to evaporate and the solution stirred for 1 h at room temperature. The reaction was worked up as in method 1 except that after treatment with acid and evaporation of tetrahydrofuran the mixture was extracted with ethyl acetate (150 ml). The ethyl acetate extract was washed with brine (20 ml), dried and evaporated. The residue was dissolved in acetone/diethyl ether. The colourless crystals which formed on cooling to 0° were collected to give the title compound (9.2) (1.67 g, 22%), m.p. 103-105° (after some softening at 95°) (Found: C, 56.3; H, 6.1; S, 12.4. $C_{12}H_{16}O_4S$ requires C, 56.2; H, 6.3; S, 12.5%). Mass spectrum (C.I.): m/z 257 $(M+1)^{+\cdot}$. $^1H$ n.m.r. δ 9.90, broad s, OH; 5.40, s, H8; 4.88-4.52, m, H3; 3.00-1.78, m, 10H; 1.26, t, J 6 Hz, $CH_3$ $_{max}$ (thin film): 1755 (s, ($C_1$=0), 1585 (s, C=0) $cm^{-1}$.

The mother liquors were evaporated and the residue subjected to column chromatography using chloroform/5% methanol as the eluant to afford more (9.2) (3.05 g, 39%).

(b) 8-Butyryl-3-(ethylthiomethyl)-9-hydroxy-2-oxaspiro[4.5]dec-8-ene-1,7-dione (10.2)

A stirred solution of the lactone (9.2) (5.49 g, 21.4 mmol) and 4-dimethylaminopyridine (523 g, 4.28 mmol) in butyric anhydride (10.50 ml, 64.2 mmol) and triethylamine (8.96 ml, 64.2 mmol) was heated to 80° for 1 h under argon and then worked up as described for the preparation of (10.1) to afford the title compound (10.2) (1.132 g, 16%) as a pale yellow oil. $^1H$ n.m.r. δ ($CDCl_3$) 18.54, s, OH; 4.92-4.52, m, H3; 3.32-1.42, m, 14H; 1.27, t, J 7 Hz, $CH_3$; 1.00, t, J 7 Hz, $CH_3$. Mass spectrum (C.I.): 327 $(M+1)^{+\cdot}$. $_{max}$ (thin film): 1765 (s, CO), 1665 (s, CO) $cm^{-1}$.

(c) 8-((1-Ethoxyimino)butyl)-3-(ethylthiomethyl)-9-hydroxy-2-oxaspiro[4.5]dec-8-ene-1,7-dione (3.30)

A solution of (10.2) (1.00 g, 3.06 mmol) and ethoxyamine hydrochloride (597 mg, 6.12 mmol) in ethanol (20 ml) and pyridine (2.5 ml) was stirred overnight at room temperature. Work up as described for the preparation of (3.3) afforded the title compound (3.30) (675 mg, 60%) as a pale yellow oil. $^1H$ n.m.r. $\delta$ ($CDCl_3$) 15.36, s, OH; 4.88-4.50, m, H3; 4.10, q, J 7 Hz, $OC\underline{H_2}CH_3$; 3.30-2.60, m, 7H; 2.62, d, J 6 Hz, $EtSC\underline{H_2}$; 2.43, q, J 6 Hz, $CH_3C\underline{H_2}S$; 2.12-1.82, m, 1H; 1.80-1.30, m, 2H; 1.32, t, J 6 Hz, $\underline{CH_3}CH_2S$; 1.26, t, J 7 Hz, $OCH_2C\underline{H_3}$, 0.98, t, J 7 Hz, $CH_3$. Mass spectrum (C.I.): m/z 370 $(M+1)^{+\cdot}$. $_{max}$ (thin film): 1765 (s, CO), 1650, (s, CO), 1600 (s) $cm^{-1}$.

EXAMPLE ~~8~~ 9

The post-emergent herbicidal activities of the compounds of the invention were assessed by the following procedure:

Seeds of each of the test species were sown 5 mm deep in presterilized soil in square plastic pots approximately 6cm x 7cm with an appropriate number of seeds per pot to avoid overcrowding and allow satisfactory plant development. The pots were then placed at randomised positions in trays 30cm x 34 cm so that each tray contained one of each test species.

All the trays were placed in a glasshouse, lightly watered with an overhead spray to initiate germination and then spray irrigated as required for optimum plant growth. After the plants had grown to a height of 10 to 12.5 cm the required quantity of the test compound was dissolved in acetone and the acetone solution dispersed in water to give a spray liquid volume equivalent to 1000 l/ha.

Two trays were sprayed with the test compound for each application rate using a flat fan even swathe nozzle. One tray for each ten chemical treatments was sprayed with acetone/water solution only and was included in the remainder of the test procedure to act as control.

The treated trays were then returned to the greenhouse. After one and three weeks post-treatment the effect of the treatment was visually assessed. The assessments were on a 0-10 scale, where 0 = no effect and 10 = plants dead.

The test species and results are shown for compounds (2.2), (3.3) and (3.7) in Tables 3, 4 and 5 respectively.

EXAMPLE ~~7~~ 10

The pre-emergent herbicidal activities of the compounds of the invention were assessed by the following procedure:

Seeds of each of the test species were sown 5 mm deep in presterilized soil in square plastic pots approximately 6cm x 7cm with an appropriate number of seeds per pot to avoid overcrowding and allow satisfactory plant development. The pots were then placed at randomised positions in trays 30cm x 34 cm so that each tray contained one of each test species.

The required quantity of the test compound was dissolved in acetone and the acetone solution dispersed in water to give a spray liquid volume equivalent to 1000 l/ha.

Two trays were sprayed with the test compound for each application rate using a flat fan even swathe nozzle. One tray for each ten chemical treatments was sprayed with acetone/water only and was included in the remainder of the test procedure to act as control. All the trays were placed in a glasshouse, lightly watered with an overhead spray to

initiate germination and then spray irrigated as required for optimum plant growth. After three weeks the trays were removed from the greenhouse and the effect of the treatment was assessed. The assessments were on a 1-10 scale, where 0 = no effect and 10 = plants dead.

The test species and results are shown for compounds (2.2), (3.3) and (3.7) in Tables 3, 4 and 5 respectively.

TABLE 3

HERBICIDAL ACTIVITY OF COMPOUND (2.2)

| PLANT | MEAN HERBICIDAL RATING | | | | | |
|---|---|---|---|---|---|---|
| | PRE-EMERGENT | | | POST-EMERGENT | | |
| FAMILY - Species | APPLICATION RATE kg/ha | | | APPLICATION RATE kg/ha | | |
| | 0 | 0.5 | 5 | 0 | 0.5 | 5 |
| MONOCOTYLEDONS | | | | | | |
| GRAMINEAE | | | | | | |
| - Echinochloa crus-galli | 0 | 2.5 | 10 | 0 | 7.5 | 9 |
| - Panicum sp. | 1 | 9.5 | 10 | 0 | 8.5 | 10 |
| - Lolium rigidum | 0 | 9.5 | 10 | 0 | 7 | 10 |
| - maize [Zea mays] | 0 | 0 | 3 | 0 | 4 | 10 |
| - rice [Oryza sativa] | 0 | 0 | 9 | 1 | 5.5 | 9 |
| - wheat [Triticum sp.] | 0 | 0 | 4 | 0 | 0 | 2.5 |
| CYPERACEAE | | | | | | |
| - cyperus rotundus | 0 | 0 | 0 | 0 | 0 | 0 |
| ALLIACEAE | | | | | | |
| - onion [Allium cepa] | 0 | 0 | 0 | 0 | 0 | 0 |
| DICOTYLEDONS | | | | | | |
| CHENOPODIACEAE | | | | | | |
| - Chenopodium album | 0 | 0 | 0 | 0 | 0 | 0 |
| AMARANTHACEAE | | | | | | |
| - Amaranthus sp. (weed) | 0 | 0 | 0 | 0 | 0 | 0 |
| - red beet [Amaranthus retroflexus] | 0 | 0 | 0 | 0 | 0 | 0 |
| CRUCIFERAE | | | | | | |
| - rapeseed [Brassica napus] | 0 | 0 | 0 | 0 | 0 | 0 |
| LEGUMINOSAE | | | | | | |
| - soy bean [Glycine max] | 0 | 0 | 0 | 0 | 0 | 0 |
| MALVACEAE | | | | | | |
| - cotton [Gossypium sp.] | 0 | 0 | 0 | 0 | 0 | 0.5 |
| SOLANACEAE | | | | | | |
| - tomato [Lycopersicon esculentum] | 0 | 0 | 0 | 0 | 0 | 0 |
| CURCURBITACEAE | | | | | | |
| - squash [Cucurbita pepo] | 0 | 0 | 2 | 0 | 0 | 0 |
| COMPOSITAE | | | | | | |
| - sunflower [Helianthus annuus] | 0 | 0 | 0 | 0 | 0 | 0 |
| - saiflower [Carthamus tinctorius] | 0.5 | 0 | 0 | 0 | 0 | 0 |

TABLE 4

HERBICIDAL ACTIVITY OF COMPOUND (3.3)

| PLANT<br>FAMILY - Species | MEAN HERBICIDAL RATING<br>PRE-EMERGENT<br>APPLICATION RATE kg/ha<br>0 | <br><br><br>0.5 | <br><br><br>5 | <br>POST-EMERGENT<br>APPLICATION RATE kg/ha<br>0 | <br><br><br>0.5 | <br><br><br>5 |
|---|---|---|---|---|---|---|
| MONOCOTYLEDONS | | | | | | |
| GRAMINEAE | | | | | | |
| - Echinochloa crus-galli | 0 | 9.5 | 10 | 1 | 9 | 10 |
| - Panicum sp. | 0 | 10 | 10 | 0 | 10 | 10 |
| - Lolium rigidum | 0 | 10 | 10 | 0 | 10 | 10 |
| - maize [Zea mays] | 0 | 6.5 | 10 | 0 | 10 | 10 |
| - rice [Oryza sativa] | 0 | 10 | 10 | 1 | 10 | 10 |
| - wheat [Triticum sp.] | 0 | 7 | 10 | 0 | 9 | 10 |
| CYPERACEAE | | | | | | |
| - cyperus rotundus | 0 | 0 | 0 | 0 | 0 | 0 |
| ALLIACEAE | | | | | | |
| - onion [Allium cepa] | 0 | 0 | 0 | 0 | 0 | 0 |
| DICOTYLEDONS | | | | | | |
| CHENOPODIACEAE | | | | | | |
| - Chenopodium album | 0 | 4.5 | 5 | 0 | 0 | 0 |
| AMARANTHACEAE | | | | | | |
| - Amaranthus sp. (weed) | 0 | 0 | 8.5 | 0 | 0 | 1 |
| - red beet [Amaranthus retroflexus] | 0 | 0 | 0 | 0 | 0 | 2 |
| CRUCIFERAE | | | | | | |
| - rapeseed [Brassica napus] | 0 | 0 | 0 | 0 | 0 | 0 |
| LEGUMINOSAE | | | | | | |
| - soy bean [Glycine max] | 0 | 0 | 0 | 0 | 0 | 0 |
| MALVACEAE | | | | | | |
| - cotton [Gossypium sp.] | 0 | 0 | 0 | 0 | 0 | 0 |
| CONVOLVULACEAE | | | | | | |
| - Datura stramonium | 0 | 0 | 0 | 0 | 0 | 1 |
| SOLANACEAE | | | | | | |
| - tomato [Lycopersicon esculentum] | 0 | 0 | 0 | 0 | 3 | 0 |
| CURCURBITACEAE | | | | | | |
| - squash [Cucurbita pepo] | 0 | 0 | 0 | 0 | 7 | 4 |
| COMPOSITAE | | | | | | |
| - sunflower [Helianthus annuus] | 0 | 0 | 0 | 0 | 0 | 0 |
| - safflower [Carthamus tinctorius] | 0 | 0 | 0 | 0 | 0 | 0 |

TABLE 5

HERBICIDAL ACTIVITY OF COMPOUND (3.7)

| PLANT | MEAN HERBICIDAL RATING | | | | | |
|---|---|---|---|---|---|---|
| | PRE-EMERGENT | | | POST-EMERGENT | | |
| FAMILY - Species | APPLICATION RATE kg/ha | | | APPLICATION RATE kg/ha | | |
| | 0 | 0.5 | 5 | 0 | 0.5 | 5 |
| MONOCOTYLEDONS | | | | | | |
| GRAMINEAE | | | | | | |
| - Echinochloa crus-galli | 1 | 9 | 10 | 0.5 | 10 | 10 |
| - Panicum sp. | 0.5 | 10 | 10 | 0 | 10 | 10 |
| - Lolium rigidum | 0 | 9.5 | 10 | 0 | 10 | 10 |
| - maize [Zea mays] | 0 | 8 | 10 | 0 | 10 | 10 |
| - rice [Oryza sativa] | 0 | 10 | 10 | 0 | 9.5 | 10 |
| - wheat [Triticum sp.] | 0 | 8 | 10 | 0 | 5.5 | 10 |
| CYPERACEAE | | | | | | |
| - cyperus rotundus | 0 | 0 | 1 | 0 | 1.5 | 2.5 |
| ALLIACEAE | | | | | | |
| - onion [Allium cepa] | 0 | 0 | 0.5 | 0.5 | 5 | 4 |
| DICOTYLEDONS | | | | | | |
| CHENOPODIACEAE | | | | | | |
| - Chenopodium album | 0 | 0.5 | 4 | 1 | 2 | 1 |
| AMARANTHACEAE | | | | | | |
| - Amaranthus sp. (weed) | - | - | - | - | - | - |
| - red beet [Amaranthus retroflexus] | 0 | 1.5 | 2 | 3 | 0 | 0 |
| CRUCIFERAE | | | | | | |
| - rapeseed [Brassica napus] | 0 | 5 | 8.5 | 0 | 3.5 | 0 |
| LEGUMINOSAE | | | | | | |
| - lucerne [Medicago satira] | 0 | 2 | 3 | 1 | 0 | 0 |
| MALVACEAE | | | | | | |
| - cotton [Gossypium sp.] | - | 2 | 0.5 | 1 | 2 | 2.5 |
| CONVOLVULACEAE | | | | | | |
| - Datura stramonium | - | - | - | - | - | - |
| SOLANACEAE | | | | | | |
| - tomato [Lycopersicon esculentum] | 0 | 3.5 | 3 | 0 | 0 | 0 |
| CURCURBITACEAE | | | | | | |
| - squash [Cucurbita pepo] | 0 | 3.5 | 5 | 0.5 | 1 | 0.5 |
| COMPOSITAE | | | | | | |
| - sunflower [Helianthus annuus] | 0 | 1.5 | 1 | - | - | - |
| - safflower [Carthamus tinctorius] | 0 | 1 | 0.5 | 0.5 | 3 | 0 |

CLAIMS :

1. Compounds of the general formula (2), including all isomeric and/or tautomeric forms thereof;

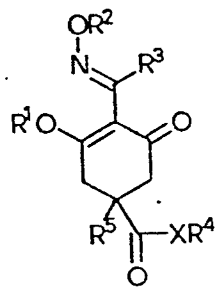

(2)

characterized in that $R^1$ is selected from the group consisting of: hydrogen; alkyl; alkenyl; alkynyl; substituted alkyl wherein the alkyl group is substituted with a substituent selected from the group consisting of alkoxy, alkylthio, optionally substituted phenyl, optionally substituted heterocycle; optionally substituted phenyl; optionally substituted heterocycle; alkyl sulfonyl; optionally substituted benzene sulfonyl; acyl and an inorganic or organic cation;

$R^2$ is selected from the group consisting of: alkyl; alkenyl; haloalkenyl; alkynyl; haloalkynyl; substituted alkyl wherein the alkyl group is substituted with a substituent selected from the group consisting of halogen, alkoxy, alkylthio, optionally substituted phenyl, and optionally substituted heterocycle; optionally substituted phenyl; and optionally substituted heterocycle;

$R^3$ is selected from the group consisting of: alkyl; fluoroalkyl; alkenyl; alkynyl; and optionally substituted phenyl;

X is O or $NR^6$

$R^4$ is selected from the group consisting of: hydrogen; alkyl; alkenyl; alkynyl; substituted alkyl wherein the alkyl group is substituted with a substituent selected from the group consisting of alkoxy, alkylthio, optionally substituted phenyl, and optionally substituted heterocycle; optionally substituted phenyl; and optionally substituted heterocycle;

$R^5$ is selected from the group consisting of: alkyl; substituted alkyl wherein the alkyl group is substituted with a substituent selected from the group consisting of alkoxy, alkylthio, optionally substituted phenyl, and optionally substituted heterocycle;

$R^6$ is selected from the group consisting of hydrogen; alkyl; substituted alkyl wherein the alkyl group is substituted with a substituent selected from the group consisting of alkoxy, alkylthio, optionally substituted phenyl, and optionally substituted heterocycle; optionally substituted phenyl; and optionally substituted heterocycle.

OR

$R^6$ and $R^4$ together with the nitrogen to which they are attached form a substituted or unsubstituted heterocyclic ring.

OR

$COXR^4$ and $R^5$ together with the carbon to which they are attached form a substituted or unsubstituted 5- or 6-membered heterocyclic ring.

2. Spirocyclic compounds of the general formula (3)

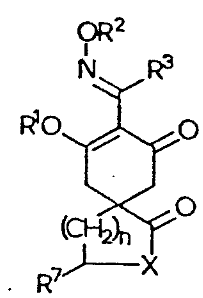

(3)

characterised in that $R^1$, $R^2$, $R^3$ and X are as specified in Claim 1, $R^7$ is selected from the group consisting of: hydrogen; alkyl; substituted alkyl wherein the alkyl group is substituted with a substituent selected from the group consisting of alkoxy, alkylthio, optionally substituted phenyl, and optionally substituted heterocycle; and n is 1 or 2.

3. Compounds as claimed in Claim 1 or Claim 2 characterized in that any heterocyclic ring present has more than four atoms.

4. Compounds as claimed in Claim 3, characterized in that the heterocyclic ring(s) is/are thiophenyl, benzofuranyl, furanyl, morpholino or pyridyl.

5. Compounds as claimed in any of the preceeding claims, characterized in that the alkyl, alkenyl, alkynyl groups are lower alkyl, alkenyl or alkynyl groups.

6. Compounds as claimed in Claim 5, characterized in that alkyl, alkoxy, alkylthio, haloalkyl, alkyl sulphonyl or substituted alkyl groups contain from 1 to 6 carbon atoms and alkenyl, alkynyl, haloalkenyl, or haloalkynyl groups contain from 2 to 6 carbon atoms.

7. Compounds as claimed in any of the preceeding claims, characterized in that $R^1$ is hydrogen or an alkali metal.

8. Compounds as claimed in any one of the preceeding claims, characterized in that $R^2$ is ethyl or allyl.

9. Compounds as claimed in any one of the preceeding claims, characterized in that $R^3$ is ethyl or n-propyl.

10. Compounds as claimed in any one of the preceeding claims, characterized in that X and n for compounds of formula (3) are O and 1 respectively.

11. A process for preparing a compound of the general formula (2) as stated and defined in Claim 1, characterized in that a compound of the general formula (8)

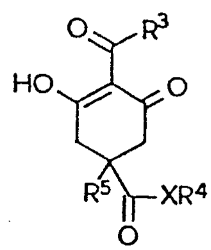

(8)

wherein $R^3$, $R^4$ and $R^5$ are as defined in Claim 1, except that $XR^4$ is not OH, is reacted with an appropriately substituted alkoxyamine to give a compound of formula (2) in which $R^1$ is hydrogen.

12. A process as claimed in Claim 11, characterized in that the compound of formula (8) is prepared by acylating a compound of formula (6) by a Fries rearrangement.

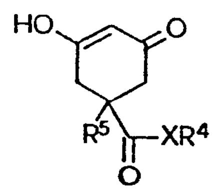

(6)

13. A process as claimed in Claim 12, characterized in that a compound of formula (4) is converted to an ester of formula (5) or amide derivative of formula (7)

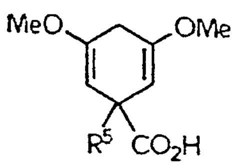

(4)

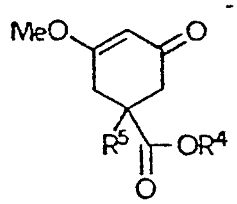

(5)

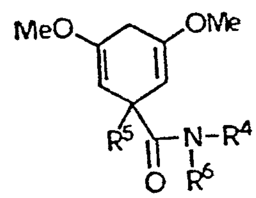

(7)

the ester or amide derivative ( 5 or 7) is treated with aqueous acid to produce the compound (6).

14. A process as claimed in Claim 13, characterized in that the compound of formula (4) is prepared by reducing 3,5-dimethoxy benzoic acid with an alkali metal in liquid ammonia and adding an electrophile to the reaction mixture after the reduction.

15. A process as claimed in any one of Claims 11 to 14, characterized in that a compound of formula (2), in which X is O and $R^4$ is other than hydrogen, is hydrolysed to give a compound in which $XR^4$ is OH.

16. A process as claimed in any of Claims 11 to 14, characterized in that the compound of formula (2) in which $R^1$ is hydrogen is reacted with an organic or inorganic salt to give a compound in which $R^1$ is an organic or inorganic cation, or the compound is

reacted with a suitable reactive derivative of one of the groups $R^1$ to produce a compound of formula (2) in which $R^1$ is other than hydrogen.

17. A process as claimed in Claim 14, for the production of a lactone derivative of formula (3) as stated and defined in Claim 2, wherein X is O and n is 1, characterized in that 3,5-dimethoxybenzoic acid is reduced by an alkali metal in liquid ammonia followed by the addition of an appropriately substituted 1,2-dihaloalkane or oxirane to produce, after acid hydrolysis, a 9-hydroxy-2-oxaspiro [4.5]dec-8-ene-1,7-dione derivative of the formula (9)

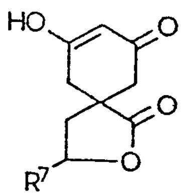

(9)

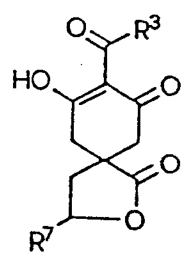

(10)

acylating the compound (9) at the 8-position by a Fries rearrangement reaction to produce a compound of formula (10), which is then reacted with an appropriate alkoxyamine to produce the compound of formula (3).

18. A plant growth inhibiting, plant damaging, or plant killing composition comprising a compound of

formula (2) as defined in Claim 1 and an inert carrier therefor.

19. A process for regulating the growth of a plant which process comprises applying to the plant, to the seed of the plant, or to the growth medium of the plant, an effective amount of a compound of formula (2) as defined in Claim 1.

20. A plant growth regulating composition comprising a compound of formula (2), as defined in Claim 1, and an inert carrier therefor.

21. The use of a compound of formula (2) as a herbicide or plant growth regulator.